# EUROPEAN PATENT SPECIFICATION

(11) **EP 2 576 830 B1**
(45) Date of publication and mention of the grant of the patent: **27.08.2014**
(21) Application number: 11731575.4
(22) Date of filing: 02.06.2011
(51) Int. Cl.: C12Q 1/68

(54) **METHODS FOR IN SITU DETECTION OF NUCLEOTIDE SEQUENCES**
VERFAHREN FÜR DEN IN-SITU-NACHWEIS VON NUKLEOTIDSEQUENZEN
PROCÉDÉS DE DÉTECTION IN SITU DE SÉQUENCES DE NUCLÉOTIDES

(30) Priority: 03.06.2010 US 351064 P
(43) Date of publication of application: 10.04.2013
(73) Proprietor: Cellay, Inc., Cambridge, Massachusetts 02139 (US)
(72) Inventor: AURICH-COSTA, Joan, Cambridge, MA 02139 (US)
(74) Representative: Potter Clarkson LLP
(86) International application number: PCT/US2011/038934
(87) International publication number: WO 2011/153354

(56) References cited:
- WO-A2-2005/007872
- US-A1- 2007 128 646
- HE H ET AL: "FLUORESCENCE IN SITU HYBRIDIZATION OF METAPHASE CHROMOSOMES IN SUSPENSION", INTERNATIONAL JOURNAL OF RADIATION BIOLOGY, TAYLOR & FRANCIS, GB, vol. 77, no. 7, 1 July 2001 (2001-07-01), pages 787-795, XP009070378, ISSN: 0955-3002
- TANKE H J ET AL: "NEW STRATEGY FOR MULTI-COLOUR FLUORESCENCE IN SITU HYBRIDISATION: COBRA: COMBINED BINARY RATIO LABELLING", EUROPEAN JOURNAL OF HUMAN GENETICS, KARGER, BASEL, CH, vol. 7, no. 1, 1 January 1999 (1999-01-01) , pages 2-11, XP000930083, ISSN: 1018-4813, DOI: 10.1038/SJ.EJHG.5200265

## Description

### BACKGROUND OF THE INVENTION

Fluorescence *in situ* hybridization (FISH) has been employed as a molecular technique to localize DNA sequences on human chromosomes for more than 20 years (Bauman 1985; Pinkel 1986). Over the past two decades, refinement of various aspects of the FISH technique has advanced the field of human cytogenetics and molecular diagnostics, allowing for the identification of chromosomal abnormalities associated with solid tumors and hematopoietic malignancies, and for the diagnosis of infectious diseases. (Heim and Mitelman 1995; Klinger 1995; Timm, Podniesinski et al. 1995; Heselmeyer, Macville et al. 1997; Sauer, Wiedswang et al. 2003).

Current FISH procedures are labor intensive and time consuming, requiring multiple manual processing steps and adherence to precise temperature and time requirements. Standard FISH techniques typically require more than a dozen steps to process a slide sample, several of which are performed at different temperatures, necessitating the use of numerous, and often costly, temperature equipment, such as water baths, hot plates, and incubators. These and other limitations of the technique have prevented it from being used more widely in research and clinical laboratories.

Presently, there is a need for more simplified and cost-effective methods of performing FISH that require fewer processing steps, less time and less equipment.

### SUMMARY OF THE INVENTION

The invention is defined in the accompanying claims.

The present invention is described with reference to the following numbered paragraphs:
1. A method for determining whether a target nucleic acid is present in a biological sample on a solid support, comprising the steps of:
   a) contacting the sample on the support with a denaturation buffer comprising a base and about 50% to about 80% alcohol, wherein the base is present in the solution at a concentration of about 0.03N to about 0.17N, thereby denaturing the sample;
   b) incubating the denatured sample in a hybridization buffer that comprises at least one single-stranded oligonucleotide probe at a temperature in the range of about 19 degrees Celsius to about 25 degrees Celsius, wherein the oligonucleotide probe comprises a nucleotide sequence that is substantially complementary to a nucleotide sequence in the target nucleic acid and at least one detectable label;
   c) washing the sample to remove unhybridised oligonucleotide probes; and
   d) determining whether the target nucleic acid is present in the sample by detecting one or more oligonucleotide probes that have hybridized to the target nucleic acid in the sample.
2. The method of paragraph 1, wherein the base is sodium hydroxide.
3. The method of paragraph 1 or 2, wherein step a) is performed at a temperature of about 19 degrees Celsius to about 25 degrees Celsius, for example at a temperature of about 21 degrees Celsius.
4. The method of paragraph 3, wherein the sample is contacted with the solution for about 3 to about 20 minutes in step a), for example for about 11 to about 17 minutes in step a), and preferably for about 13 to about 15 minutes in step a).
5. The method of paragraph 3, wherein the solution in step a) comprises about 0.07M sodium hydroxide.
6. The method of paragraph 5, wherein the solution in step a) comprises about 70% ethanol.
7. The method of paragraph 1 or 2, wherein step b) is performed at a temperature of about 21 degrees Celsius.
8. The method of paragraph 1, wherein, prior to step b), the oligonucleotide probe is in a hybridization buffer that includes formamide, dextran sulfate, and one or more salts at a final concentration of about 0.03M to about 0.09M.
9. The method of paragraph 1, wherein the step of removing unhybridised oligonucleotide probes from the sample comprises washing the sample in a wash buffer at a temperature of about 19 degrees Celsius to about 25 degrees Celsius prior to step c).
10. The method of paragraph 9, wherein the wash buffer includes one or more salts at a final concentration of about 0.03M to about 0.09M, and sodium dodecyl sulfate (SDS).
11. The method of paragraph 1, wherein the oligonucleotide probe comprises about 20 to about 50 nucleotides, for example about 30 nucleotides.
12. The method of paragraph 11, wherein the oligonucleotide probe is a synthetic oligonucleotide probe.
13. The method of paragraph 1, wherein the at least one detectable label is attached to the oligonucleotide by a covalent bond.
14. The method of paragraph 13, wherein the at least one detectable label comprises a fluorescent label.
15. A method for detecting a target nucleic acid in a biological sample on a solid support, comprising the steps of:
   a) contacting the sample on the support with a denaturation buffer comprising a base and about 50% to about 80% alcohol, wherein the base is present in the solution of step a) at a concentration of about 0.03N to about 0.17N, thereby denaturing the sample;
   b) hybridizing at least one single-stranded oligonucleotide probe to the target nucleic acid in the denatured sample in a hybridization buffer at a temperature in the range of about 19 degrees Celsius to about 25 degrees Celsius, wherein the oligonucleotide probe comprises a nucleotide sequence that is substantially complementary to a nucleotide sequence in the target nucleic acid and at least one detectable label;
   c) washing the sample to remove unhybridised oligonucleotide probes; and
   d) detecting the at least one detectable label on the oligonucleotide probe following hybridization to the target nucleic acid in the sample, thereby detecting the target nucleic acid in the sample.
16. The method of paragraph 15, wherein the base is sodium hydroxide.
17. The method of paragraph 1 or 15, wherein the biological sample comprises urothelial cells.
18. A kit for detecting a target nucleic acid in a biological sample, comprising:
   a) at least one single-stranded oligonucleotide probe consisting of about 20 to about 50 nucleotides, wherein at least one detectable label is covalently attached to the oligonucleotide probe;
   b) a denaturation buffer comprising about 0.03M to about 0.17M sodium hydroxide and about 50% to about 80% alcohol;
   c) a hybridization buffer comprising about 20% to about 90% formamide, dextran sulfate, and one or more salts at a final concentration of about 0.03M to about 0.09M; and
   d) a wash buffer that includes one or more salts at a final concentration of about 0.03M to about 0.09M, and about 0.1 % SDS.
19. The kit of paragraph 18, wherein the denaturation buffer includes about 0.07M sodium hydroxide and about 70% ethanol.
20. The kit of paragraph 18 or 19, wherein the hybridization buffer includes about 60% to about 80% formamide.
21. The kit of paragraph 18, wherein the one or more salts in the wash buffer are selected from the group consisting of a sodium salt, a lithium salt and a potassium salt.
22. The kit of paragraph 18, wherein the one or more salts in the wash buffer include sodium citrate and sodium chloride.
23. The kit of paragraph 21 or 22, wherein the wash buffer further includes formamide.
24. A method for detecting a target nucleic acid in a biological sample on a solid support, comprising the steps of:
   a) contacting the sample on the support with a denaturation buffer comprising about 0.07M sodium hydroxide and about 70% ethanol for about 13 to about 15 minutes at a temperature in the range of about 19 degrees Celsius to about 25 degrees Celsius, thereby denaturing the sample;
   b) hybridizing at least one single-stranded oligonucleotide probe consisting of about 20 to about 50 nucleotides to the target nucleic acid in the denatured sample in a hybridization buffer at a temperature in the range of about 19 degrees Celsius to about 25 degrees Celsius, wherein the oligonucleotide probe comprises a nucleotide sequence that is substantially complementary to a nucleotide sequence in the target nucleic acid, and at least one fluorescent detectable label covalently attached to the oligonucleotide probe;
   c) washing the sample to remove unhybridised oligonucleotide probes; and
   d) detecting the fluorescent detectable label on the oligonucleotide probe, thereby detecting the target nucleic acid in the sample.

All steps in the methods of the invention, unless otherwise specified, can be performed at room temperature, obviating the need for expensive temperature equipment and adherence to precise and variable temperature requirements. The methods of the invention also require fewer steps and less time to complete than standard FISH techniques.

### BRIEF DESCRIPTION OF THE DRAWINGS

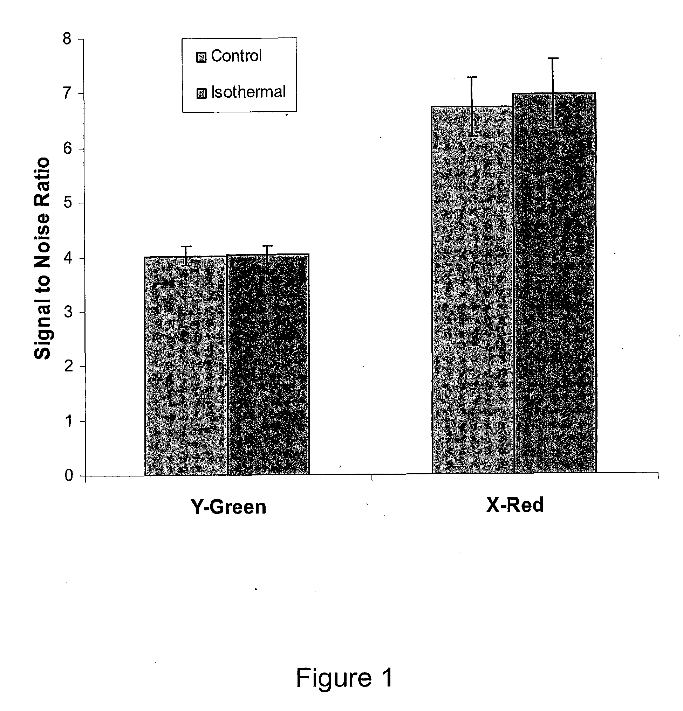

Figure 1 is a graph depicting a comparison of signal-to-noise ratios produced by labeled Y- and X-chromosome oligonucleotide probes following FISH using conventional denaturation conditions (blue) or room temperature denaturation conditions (grey). n=50, ± SEM (standard error of the mean).

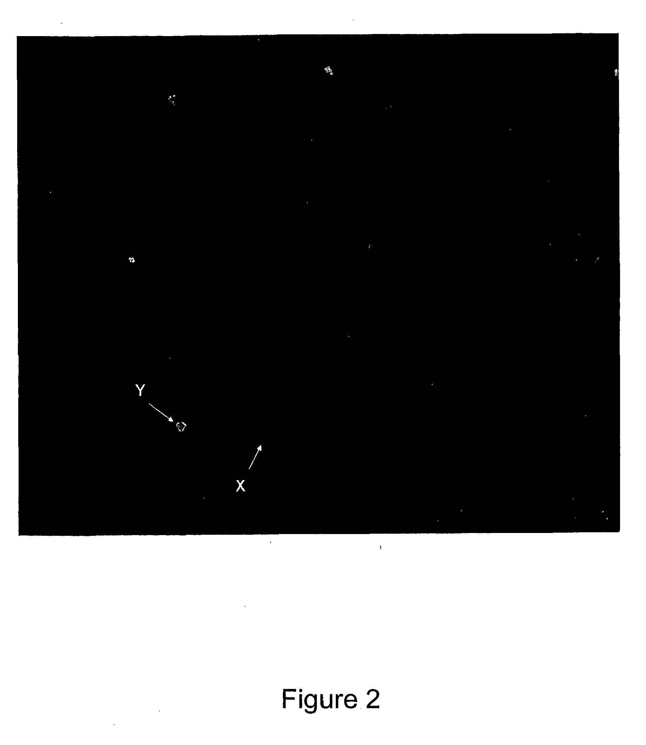

Figure 2 is an image of a metaphase chromosome spread and interphase nuclei from peripheral blood showing signals produced by Oligo-FISH(TM) X- (red) and Y-chromosome (green) (arrows) probes following FISH using room temperature denaturation and hybridization steps and standard wash conditions (0.2X SSC, 0.1 % SDS, at 50°C). Image magnification is 600X.

### DETAILED DESCRIPTION OF THE INVENTION

### Definitions

Unless defined otherwise, all technical and scientific terms used herein have the same meaning as commonly understood by one of ordinary skill in the art to which this invention pertains.

As used herein, the terms "room temperature" or "RT" refer to temperatures in the range of about 18 degrees Celsius to about 25 degrees Celsius.

The term "isothermal" refers to consistent or constant temperatures. For example, denaturation and hybridization steps that are both performed at room temperature are referred to as isothermal denaturation and hybridization conditions.

The term "nucleotide" refers to naturally occurring ribonucleotide or deoxyribonucleotide monomers, as well as non-naturally occurring derivatives and analogs thereof. Accordingly, nucleotides can include, for example, nucleotides comprising naturally occurring bases *(e.g.,* A, G, C, or T) and nucleotides comprising modified bases (*e.g.,* 7-deazaguanosine, or inosine).

The term "sequence," in reference to a nucleic acid, refers to a contiguous series of nucleotides that are joined by covalent bonds *(e.g.,* phosphodiester bonds).

The term "nucleic acid" refers to a polymer having multiple nucleotide monomers. A nucleic acid can be single- or double-stranded, and can be DNA *(e.g.,* cDNA or genomic DNA), RNA, or hybrid polymers (*e.g.,* DNA/RNA). Nucleic acids can be chemically or biochemically modified and/or can contain non-natural or derivatized nucleotide bases. Nucleic acid modifications include, for example, methylation, substitution of one or more of the naturally occurring nucleotides with an analog, internucleotide modifications such as uncharged linkages (*e.g.,* methyl phosphonates, phosphotriesters, phosphoamidates, carbamates, and the like), charged linkages (*e.g.,* phosphorothioates, phosphorodithioates, and the like), pendent moieties *(e.g.,* polypeptides), intercalators *(e.g.,* acridine, psoralen, and the like), chelators, alkylators, and modified linkages (*e.g.,* alpha anomeric nucleic acids, and the like). Nucleic acids also include synthetic molecules that mimic nucleic acids in their ability to bind to a designated sequence via hydrogen bonding and other chemical interactions. Typically, the nucleotide monomers are linked via phosphodiester bonds, although synthetic forms of nucleic acids can comprise other linkages (*e.g.,* peptide nucleic acids (also referred to herein as "PNAs"), such as described in Nielsen et al., Science 254, 1497-1500, 1991). Nucleic acids can also include, for example, conformationally restricted nucleic acids (*e.g.,* "locked nucleic acids" or "LNAs," such as described in Nielsen et al., J. Biomol. Struct. Dyn. 17:175-91, 1999), morpholinos, glycol nucleic acids (GNA) and threose nucleic acids (TNA). "Nucleic acid" does not refer to any particular length of polymer and can, therefore, be of substantially any length, typically from about six (6) nucleotides to about 10⁹ nucleotides or larger. In the case of a double-stranded polymer, "nucleic acid" can refer to either or both strands.

The term "oligonucleotide" refers to a short nucleic acid, typically about 6 to about 100 nucleotide bases in length, joined by covalent linkages, such as phosphorus linkages (*e.g.,* phosphodiester, alkyl and aryl-phosphonate, phosphorothioate, phosphotriester), and/or non-phosphorus linkages (*e.g.,* peptide, sulfamate, and others).

The term "target nucleic acid" refers to a nucleic acid whose presence or absence in a sample is desired to be detected.

The term "target sequence" refers to a nucleotide sequence in a target nucleic acid that is capable of forming a hydrogen-bonded duplex with a complementary sequence (*e.g.,* a substantially complementary sequence) on an oligonucleotide probe.

As used herein, "complementary" refers to sequence complementarity between two different nucleic acid strands or between two regions of the same nucleic acid strand. A first region of a nucleic acid is complementary to a second region of the same or a different nucleic acid if, when the two regions are arranged in an anti-parallel fashion, at least one nucleotide residue of the first region is capable of base pairing *(i.e.,* hydrogen bonding) with a residue of the second region, thus forming a hydrogen-bonded duplex.

The term "substantially complementary" refers to two nucleic acid strands *(e.g.,* a strand of a target nucleic acid and a complementary single-stranded oligonucleotide probe) that are capable of base pairing with one another to form a stable hydrogen-bonded duplex under stringent hybridization conditions, including the isothermal hybridization conditions described herein. In general, "substantially complementary" refers to two nucleic acids having at least 70%, for example, about 75%, 80%, 85%, 90%, 95%, 97%, 98%, 99% or 100% complementarity.

"Repeat sequence" or "repetitive sequence" refers to noncoding tandemly repeated nucleotide sequences in the human genome including, *e.g.,* repeat sequences from the alpha satellite, satellite 1, satellite 2, satellite 3, the beta satellite, the gamma satellite and telomeres. Repeat sequences are known in the art and are described in *e.g.,* (Allshire et al., Nucleic Acids Res 17(12): 4611-27 (1989); Cho et al., Nucleic Acids Res 19(6): 1179-82 (1991); Fowler et al., Nucleic Acids Res 15(9): 3929 (1987); Haaf et al., Cell 70(4): 681-96(1992); Lee et al., Chromosoma 109(6): 381-9 (2000); Maeda and Smithies, Annu Rev Genet 20: 81-108 (1986); Meyne and Goodwin, Chromosoma 103(2): 99-103 (1994); Miklos (1985). Localized highly repetitive DNA sequences in vertebrate genomes. Molecular evolutionary genetics. I. J. R. Macintyre. NY, Plenum Publishing Corp.: 241-321 (1985); Tagarro et al., Hum Genet 93(2): 125-8 (1994); Waye and Willard, PNAS USA 86(16): 6250-4 (1989); and Willard and Waye, J Mol Evol 25(3): 207-14 (1987). The repeat sequences are located at, *e.g.,* the centromeric, pericentromeric, heterochromatic, and telomeric regions of chromosomes. Consensus repeat sequences are described in, *e.g.* Willard and Waye, J Mol Evol 25(3): 207-14 (1987) and Tagarro et al., Hum Genet 93(2): 125-8 (1994). Vissel and Choo, Nucleic Acids Res. 15(16): 6751-6752 (1987), Cho et al., Nucleic Acids Res 19(6): 1179-82 (1991).

The term "chromosome-specific nucleic acid sequence," or "chromosome-specific nucleotide sequence," as used herein, refers to a nucleic acid sequence that is specific to a particular chromosome within the genome of a cell.

The term "probe" refers to an oligonucleotide that includes a target-binding region that is substantially complementary to a target sequence in a target nucleic acid and, thus, is capable of forming a hydrogen-bonded duplex with the target nucleic acid. Typically, the probe is a single-stranded probe, having one or more detectable labels to permit the detection of the probe following hybridization to its complementary target.

As used herein, "target-binding region" refers to a portion of an oligonucleotide probe that is capable of forming a hydrogen-bonded duplex with a complementary target nucleic acid.

The term "detectable label," as used herein, refers to a moiety that indicates the presence of a corresponding moelcule (*e.g.,* probe) to which it is bound.

An "indirect label" refers to a moiety, or ligand, that is detected using a labeled secondary agent, or ligand-binding partner, that specifically binds to the indirect label.

A "direct label" refers to a moiety that is detectable in the absence of a ligand-binding partner interaction.

The term "biological sample" refers to a material of biological origin (*e.g.,* cells, tissues, organs, fluids).

A "linker," in the context of attachment of two molecules (whether monomeric or polymeric), means a molecule (whether monomeric or polymeric) that is interposed between and adjacent to the two molecules being attached. A "linker" can be used to attach, *e.g.,* oligonucleotide probe sequence and a label *(e.g.,* a detectable label). The linker can be a nucleotide linker (*i.e.,* a sequence of the nucleic acid that is between and adjacent to the non-adjacent sequences) or a non-nucleotide linker.

The term "hybrid" refers to a double-stranded nucleic acid molecule formed by hydrogen bonding between complementary nucleotides.

The term "stringency" refers to hybridization conditions that affect the stability of hybrids, *e.g.,* temperature, salt concentration, pH, formamide concentration, and the like. These conditions are empirically optimized to maximize specific binding, and minimize nonspecific binding, of a probe to a target nucleic acid.

The term "fluorophore" refers to a chemical group having fluorescence properties.

The term "optionally" means that the recited step (*e.g.,* in the case of methods of the invention) or component (*e.g.,* in the case of kits of the invention) may or may not be included.

The present invention is based, in part, on the discovery of a simplified and effective alternative fluorescence *in situ* hybridization (FISH) technique, referred to herein as "isothermal FISH," wherein sample denaturation, probe hybridization and washes can be performed at room temperature. A comparison between an exemplary conventional FISH method and an exemplary isothermal FISH method of the invention is shown in Table 1. Listed are the different steps needed for completion of the methods, along with the required temperature, apparati, and times needed for use with oligonucleotide probes. The conventional FISH technique in Table 1 requires 14 steps and approximately 133 minutes for completion, while the isothermal method of the invention requires only 4 steps and approximately 35 minutes for completion. The isothermal method of the invention also obviates the need for expensive precision temperature equipment (e.g., water baths, hotplates, incubators, freezer units) that are typically required for conventional FISH methods.

**Table 1. Comparison of Exemplary Conventional FISH and Isothermal FISH Methods.**

| **Conventional (Thermal) FISH** | | | | **Isothermal FISH** | | | |
|---|---|---|---|---|---|---|---|
| **Treatment** | **Temp. (°C)** | **Temp. Control Apparatus** | **Time (min)** | **Treatment** | **Temperature (°C)** | **Temp. Control Apparatus** | **Time (min)** |
| **RNase** | **37** | **Hot plate incubator** | **30** | **Denaturation/pretreatment NaOH/70 % Alcohol** | **RT** | **N/A** | **15** |
| **Wash** | **RT** | | **15** | | | | |
| **Protease** | **37** | **Water bath** | **5** | | | | |
| **Wash** | **RT** | | **15** | | | | |
| **1% Formaldehyde** | **RT** | | **10** | | | | |
| **Wash** | **RT** | | **5** | | | | |
| **Ethanol gradient** | **RT** | | **10** | | | | |
| **Air dry** | | | **I 5** | | | | |
| **Denaturation** | **72** | **Water bath** | **3** | | | | |
| **Ethanol gradient** | **4** | **Ice** | **10** | | | | |
| **Air dry** | **RT** | | **5** | | | | |
| **Hybridization** | **37** | **Hot plate incubator** | **5** | **Hybridization** | **RT** | **N/A** | **5** |
| **Wash** | **50** | **Water bath** | **5** | **Wash** | **RT** | **N/A** | **5** |
| **Mounting slide** | **RT** | **10** | **10** | **Mounting slide** | **RT** | **N/A** | **10** |
| **Total time (min)** | | | **133** | | | | **35** |

| | | | | | | | |
|---|---|---|---|---|---|---|---|
| RT = room temperature | | | | | | | |

Methods for Detecting a Target Nucleic Acid. The present invention provides, in one embodiment, a method for determining whether a target nucleic acid is present in a biological sample on a solid support, comprising the steps of contacting the sample on the support with a denaturation buffer comprising a base and about 50% to about 80% alcohol; wherein the base is present in the solution at a concentration of about 0.03N to about 0.17N; incubating the denatured sample in a hybridisation buffer that comprises at least one single-stranded oligonucleotide probe at a temperature in the range of about 19 degrees Celsius to about 25 degrees Celsius, wherein the oligonucleotide probe comprises a nucleotide sequence that is substantially complementary to a nucleotide sequence in the target nucleic acid and at least one detectable label; washing the sample to remove unhybridised oligonucleotide probes and determining whether the target nucleic acid is present in the sample by detecting one or more oligonucleotide probes that have hybridized to the target nucleic acid in the sample.

In another embodiment, the invention relates to a method for detecting a target nucleic acid in a biological sample; wherein the method comprises the steps of contacting the sample with a solution comprising a base and an alcohol, wherein the base is about 0.03M to about 0.17M sodium hydroxide and the alcohol is about 50% to about 80% of the solution; hybridizing at least one single-stranded oligonucleotide probe (e.g., a single stranded probe consisting of about 20 to about 50 nucleotides) that comprises at least one detectable label (e.g., a fluorophore) to the target nucleic acid in the sample at a temperature in the range of about 19 degrees Celsius to about 25 degrees Celsius; washing the sample to remove unhybridised oligonucleotide probes; and detecting the detectable label on the oligonucleotide probe, thereby detecting the target nucleic acid in the sample.

In a preferred embodiment, the invention relates to a method for detecting a target nucleic acid in a biological sample on a solid support, comprising the steps of contacting the sample on the support with a denaturation buffer comprising about 0.07M sodium hydroxide and about 70% ethanol for about 13 to about 15 minutes at a temperature in the range of about 19 degrees Celsius to about 25 degrees Celsius, thereby denaturing the sample; hybridizing a synthetic single-stranded oligonucleotide probe comprising at least one fluorescent detectable label to the target nucleic acid in the denatured sample in a hybridisation buffer at a temperature of about 19 degrees Celsius to about 25 degrees Celsius; washing the sample to remove unhybridised oligonucleotide probes; and detecting the fluorescent detectable label on the oligonucleotide probe, thereby detecting the target nucleic acid in the sample.

In another preferred embodiment, the steps of the methods of then invention are carried out entirely under isothermal conditions (e.g., at a temperature of about 21°C).

A detailed description of the various steps of the methods of the invention are set forth herein below.

### Sample Preparation/Pre-treatment

Suitable biological samples for the methods of the invention include, for example, cells (e.g., cell lines), tissues, organs, blood, spinal fluid, lymph fluid, tears, saliva, sputum, urine, semen, amniotic fluid, hair, skin, tumors (*e.g.,* a biopsy). Preferably, the biological sample includes chromosomal DNA. In a particular embodiment, the biological sample employed in the methods of the invention includes urothelial cells (*e.g.,* human urothelial cells). Preferably, the biological sample is obtained from a human.

A biological sample can include, in one embodiment, a single target nucleic acid or, in alternative embodiments, multiple target nucleic acids (*e.g.,* two or more distinct target nucleic acids). Target nucleic acids can be DNA or RNA and can include intragenic, intergenic and/or transgenic nucleotide sequences. Thus, target nucleic acids can be endogenous genomic nucleotide sequences or artificial or foreign (*e.g.,* transgenic) nucleotide sequences. Typically, a target nucleic acid comprises a chromosome-specific nucleotide sequence. Exemplary chromosome-specific nucleotide sequences are shown in Table 2.

**Table 2. Exemplary Chromosome-Specific Nucleic Acid Sequences.**

| **SEQ ID NO:** | **NAME** | **SEQUENCE (5'-3')** |
|---|---|---|
| 1 | Y1 | CCAGTCGAATCCATTCGAGTACATACC |
| 2 | Y2 | CCTTTTGAATCCATTCCATTGGAGTCC |
| 3 | Y3 | ATTCATTGCATTCCGTTTCATGAAATTCGA |
| 4 | Y4 | CTGCATACAATTTCACTCCATTCGTTCCCA |
| 5 | Y5 | TCCATTGGAGTCAATTCCTTTCGACACCCA |
| 6 | Y6 | TTGATCCTATTTTATTAAATTGCATTCTAT |
| 7 | 2.1.1 | GTGCGCCCTCAACTAACAGTGTTGAAGCTT |
| 8 | 2.2.2 | GAAACGGGATTGTCTTCATATAAACTCTAG |
| 9 | 2.5.1 | GTATCTTCCAATAAAAGCTAGATAGAAGCA |
| 10 | 2.6.1 | ATGTCAGAAACTTTTTCATGATGTATCTAC |
| 11 | 2.7.3 | TATGTGTGATGTGCGCCCTCAACTAAGAGT |
| 12 | 2.8.4 | TCTCAGAAGCTTCATTGGGATGTTTCAATT |
| 13 | 2.10.1 | GGAATACGGTGATAAAGGAAATATCTTCCA |
| 14 | 4.3.2 | TCTTTGTGTTGTGTGTACTCATGTAACAGT |
| 15 | 4.6.2 | TTTCTGCCCTACCTGGAAGCGGACATTTCG |
| 16 | 4.7.5 | GGTTATCTTCATATAAAATCCAGACAGGAG |
| 17 | 4.10.2 | CGGCACTACCTGGAAGTGGATATTTCGAGC |
| 18 | 4.18.7 | TCTGCACTACCTGGAAGAGGCCATTTCGAG |
| 19 | 4.22.10 | CCTACGGGGAGAAAGGAAATATCTTCAAAT |

Target nucleic acids can include unique or repetitive nucleotide sequences. Preferably, the target nucleic acid includes a repetitive genomic sequence, for example, a repeat sequence of a specific human chromosome (i.e., chromosome 1, 2, 3, 4, 5, 6, 7, 8, 9, 10, 11, 12, 13, 14, 15, 16, 17, 18, 19, 20, 21, 22, the X chromosome or the Y chromosome). Suitable repeat sequences include, but are not limited to, a centromeric repeat sequence, a pericentromeric repeat sequence, a heterochromatin repeat sequence, a telomeric repeat sequence, an alpha satellite repeat sequence, a beta satellite repeat sequence, a gamma satellite repeat sequence, and a satellite 1, 2, or 3 repeat sequence. In some embodiments, the target nucleic acid includes a target sequence of about 20 to about 50 contiguous nucleotides within a specific repeat sequence.

Typically, the biological sample employed in the methods of the invention is a fixed sample (e.g., a fixed cell sample, a fixed tissue sample, a chromosome spread). A variety of suitable fixatives are known in the art and include, for example, acid acetone solutions, various aldehyde solutions (e.g., formaldehyde, paraformaldehyde, and glutaraldehyde) and acid alcohol solutions. Examples of specific fixatives for chromosomal preparations are discussed, for example, in Trask et al. (Science 230: 1401-1402, 1985). The biological sample can be prepared (e.g., fixed) in solution, or on a solid support, such as, but not limited to, a microscope slide, a coverslip and a multiwell plate (e.g., a microtitre plate).

According to the invention, a biological sample is contacted with (e.g., is denatured in) a solution comprising at least one base (e.g., NaOH) at a concentration of about 0.03N to about 0.17N and about 50% to about 80% of at least one alcohol (e.g., ethanol) prior to incubating a probe with the sample. By contacting the sample with the solution comprising the base and alcohol, the nucleic acids in the sample become denatured, rendering the target nucleic acid more accessible to a complementary probe. In certain types of biological samples (e.g., sperm cells), the solution comprising the base and alcohol may also decondense the chromosomes in the sample, further promoting accessibility of a target nucleic acid to a complementary probe.

Suitable bases for use in the methods of the invention include, without limitation, potassium hydroxide, barium hydroxide, caesium hydroxide, sodium hydroxide, strontium hydroxide, calcium hydroxide, lithium hydroxide, rubidium hydroxide, magnesium hydroxide, butyl lithium, lithium diisopropylamide, lithium diethylamide, sodium amide, sodium hydride, lithium bis(trimethylsilyl)amide, sodium carbonate and ammonia, or a combination thereof. Preferably, the base is an alkali base. More preferably, the base is sodium hydroxide. Suitable concentrations of base in the base/alcohol solution employed in the methods of the invention are typically in the range of about 0.03 normal (N) to about 0.17N, for example, about 0.05N, about 0.06N, about 0.07N, about 0.08N, about 0.09N or about 0.1N. In a particular embodiment, the solution comprises about 0.07N NaOH, which is equivalent to 0.07M NaOH.

Exemplary alcohols for use in the methods of the invention include, for example, ethanol, methanol, propanol, butanol, pentanol and isoamyl alcohol, among others, or mixtures thereof. In a particular embodiment, the solution comprises ethanol. Preferably, the base/alcohol solution comprises about 0.07N base and about 70% ethanol. The alcohol can be present in the solution at a concentration of about 50% to about 90% by volume, for example about 60%, about 70% or about 80% by volume. Preferably, the alcohol is present at a concentration of about 70% by volume.

In the methods of the invention, the biological sample is typically contacted with the base/alcohol solution for a time period ranging from about 3 minutes to about 20 minutes, preferably about 11 minutes to about 17 minutes, more preferably about 13 minutes to about 15 minutes. In a particular embodiment, the sample is incubated in the base/alcohol solution at a temperature in the range of about 19° C to about 25°C, preferably about 20° C to about 22°C, more preferably about 21°C.

The methods of the invention can optionally include one or more additional steps generally employed in conventional *in situ* hybridization procedures to make nucleic acids in a sample more accessible to probes (*e.g*., pretreatment steps). Such steps include, for example, treating a biological sample with one or more proteinases (*e.g.,* proteinase K, trypsin, pepsin) and/or mild acids (*e.g.,* 0.02-0.2 N HCl, 25% to 75% acetic acid). An optional pretreatment with RNase can also be utilized to remove residual RNA from the biological sample. Other optional pre-treatment steps include fixation with formaldehyde or paraformaldehyde, detergent permeabilization, heat denaturation and aging of the sample.

### Probe Hybridization

The methods of the invention further comprise the step of incubating at least one single-stranded oligonucleotide probe to a target nucleic acid with the sample at a temperature in the range of about 19°C to about 25°C under conditions suitable for hybridizing the probe to the target nucleic acid when the target nucleic acid is present in the sample. For example, hybridization can be performed at a temperature in the range of about 19°C to about 22°C, more preferably about 21°C. Generally, hybridization is performed under conditions (e.g., temperature, incubation time, salt concentration, etc.) sufficient for a probe to hybridize with a complementary target nucleic acid in a biological sample. Suitable hybridization buffers and conditions for *in situ* hybridization techniques are generally known in the art. (See, e.g., Sambrook and Russell, supra; Ausubel *et al.,* supra. See also Tijssen, Laboratory Techniques in Biochemistry and Molecular Biology, Vol. 24: Hybridization with Nucleic Acid Probes (Elsevier, NY 1993)). For example, a hybridization buffer comprising formamide, dextran sulfate and saline sodium citrate (SSC) can be employed in the methods of the invention. Suitable concentrations of formamide in the hybridization buffer include, for example, concentrations in the range of about 20% to about 90% by volume, e.g., about 60%, about 70%, or about 80% by volume. Suitable concentrations of dextran sulfate in a hybridization buffer include, for example, about 3% to about 20%. Suitable concentrations of SSC in a hybridization buffer include, for example, about 0.1X to about 4.0X. The concentration of total salt in the hybridization buffer is preferably in the range of about 0.03M to about 0.09M.

Optimal hybridization conditions for a given target sequence and its complementary probe will depend upon several factors such as salt concentration, incubation time, and probe concentration, composition, and length, as will be appreciated by those of ordinary skill in the art. Based on these and other known factors, suitable binding conditions can be readily determined by one of ordinary skill in the art and, if necessary, optimized for use in accordance with the present methods. Typically, hybridization is carried out under stringent conditions that allow specific binding of substantially complementary nucleotide sequences.

Stringency can be increased or decreased to specifically detect target nucleic acids having 100% complementarity or to detect related nucleotide sequences having less than 100% complementarity (*e.g.,* about 70% complementarity, about 80% complementarity, about 90% complementarity). Factors such as the length and nature (DNA, RNA, base composition) of the probe sequence, nature of the target nucleotide sequence (DNA, RNA, base composition, presence in solution or immobilization) and the concentration of salts and other components in the hybridization buffer (*e.g.,* the concentration of formamide, dextran sulfate, polyethylene glycol and/or salt) in the hybridization buffer/solution can be varied to generate conditions of either low, medium, or high stringency. These conditions can be varied based on nucleotide base composition and length and circumstances of use, either empirically or based on formulas for determining such variation (see, *e.g.,* Sambrook et al., supra; Ausubel et al., supra).

In certain embodiments, a population (*e.g.,* cocktail) of two or more probes are incubated with a sample. Generally, the probe cocktail composition includes a plurality of different labeled probes, each different labeled probe having (a) a different chromosome-specific sequence and (b) a different detectable label that is distinguishable from the detectable labels on the other probes in the cocktail that are specific for a different chromosome. In some embodiments, the detectable labels on the probes are fluorophores having spectrally distinguishable emission wavelengths. Through the use of different probes labeled with distinguishable markers, such as spectrally distinguishable fluorophores, combinations of probes can be employed at the same time in order to examine the presence or absence of two or more target nucleic acids *(e.g.,* on two or more different chromosomes) in a sample. Hybridization and washing conditions can be adjusted as appropriate for differing detectable markers.

Probes that are useful in the methods of the invention comprise a target binding region consisting of a nucleotide sequence that is substantially complementary to a nucleotide sequence (*e.g.,* a target sequence) in a target nucleic acid in the sample. Although generally desirable, a target binding region in a probe is not required to have 100% complementarity to the target nucleic acid. For example, in some embodiments, probes useful in the methods of the invention can comprise a nucleotide sequence that is at least about 70%, e.g. , about 80%, about 90%, about 95% or about 99%, complementary to a nucleotide sequence in a target nucleic acid.

In a particular embodiment, the probes used in the present invention are single-stranded oligonucleotide probes (e.g., single stranded DNA oligonucleotide probes). Typical oligonucleotide probes useful in the methods of the invention are linear and range in size from about 20 to about 100 nucleotides, preferably, about 30 to about 50 nucleotides. In a particular embodiment, oligonucleotide probes that are about 30 nucleotides in length are employed in the methods of the invention.

Suitable probes for use in the methods of the invention include, but are not limited to, DNA probes, RNA probes, peptide nucleic acid (PNA) probes, locked nucleic acid (LNA) probes, morpholino probes, glycol nucleic acid (GNA) probes and threose nucleic acids (TNA) probes. Such probes can be chemically or biochemically modified and/or may contain non-natural or derivatized nucleotide bases. For example, a probe may contain modified nucleotides having modified bases (e.g., 5-methyl cytosine) and/or modified sugar groups (e.g., 2'O-methyl ribosyl, 2'O-methoxyethyl ribosyl, 2'-fluoro ribosyl, 2'-amino ribosyl). Although linear probes are preferred, useful probes can be circular or branched and/or include domains capable of forming stable secondary structures (e.g., stem- and-loop and loop-stem-loop hairpin structures).

Methods of producing probes useful in the methods of the invention are well known in the art and include, for example, biochemical, recombinant, synthetic (e.g., chemical synthesis) and semi-synthetic methods. In one embodiment, the oligonucleotide probes employed in the methods of the invention are produced by chemical synthesis. A synthetic oligonucleotide probe can be produced using known methods for nucleic acid synthesis (see, e.g., Glick and Pasternak, Molecular Biotechnology: Principles and Applications of Recombinant DNA (ASM Press 1998)). For example, solution or solid-phase techniques can be used. Synthesis procedures are typically automated and can include, for example, phosphoramidite, phosphite triester, H-phosphate, or phosphotriester methods.

Probes useful in the methods of the invention can further comprise one or more detectable labels. Labels suitable for use according to the present invention are known in the art and generally include any molecule that, by its chemical nature, and whether by direct or indirect means, provides an identifiable signal allowing detection of the probe. Thus, for example, probes may be labeled in a conventional manner, such as with specific reporter molecules, fluorophores, radioactive materials, or enzymes (*e.g.,* peroxidases, phosphatases). In a particular embodiment, the probes employed in the methods of the invention include one or more fluorophores as detectable labels.

Detectable labels suitable for attachment to probes can be indirect labels or direct labels. Exemplary indirect labels include, *e.g.,* haptens, biotin, or other specifically bindable ligands. For indirect labels, the ligand-binding partner typically has a direct label, or, alternatively, is also labeled indirectly. Examples of indirect labels that are haptens include dinitrophenol (DNP), digoxigenin, biotin, and various fluorophores or dyes (*e.g.,* fluorescein, DY490, DY590, Alexa 405/Cascade blue, Alexa 488, Bodiby FL, Dansyl, Oregon Green, Lucifer Yellow, Tetramethylrhodamine/ Rhodamine Red, and Texas Red). As an indirect label, a hapten is typically detected using an anti-hapten antibody as the ligand-binding partner. However, a hapten can also be detected using an alternative ligand-binding partner (*e.g.,* in the case of biotin, anti-biotin antibodies or streptavidin, for example, can be used as the ligand-binding partner). Further, in certain embodiments, a hapten can also be detected directly (*e.g.,* in the case of fluorescein, an anti-fluorescein antibody or direct detection of fluorescence can be used).

Exemplary "direct labels" include, but are not limited to, fluorophores (*e.g.,* fluorescein, rhodamine, Texas Red, phycoerythrin, Cy3, Cy5, DY fluors (Dyomics GmbH, Jena, Germany) Alexa 532, Alexa 546, Alexa 568, or Alexa 594). Other direct labels can include, for example, radionuclides (*e.g.,* 3H, 35S, 32P, 125I, and 14C), enzymes such as, *e.g.,* alkaline phosphatase, horseradish peroxidase, or β-galactosidase, chromophores *(e.g.,* phycobiliproteins), luminescers *(e.g.,* chemiluminescers and bioluminescers), and lanthanide chelates (*e.g.,* complexes of Eu3+ or Tb3+). In the case of fluorescent labels, fluorophores are not to be limited to single species organic molecules, but include inorganic molecules, multimolecular mixtures of organic and/or inorganic molecules, crystals, heteropolymers, and the like. For example, CdSe-CdS core-shell nanocrystals enclosed in a silica shell can be easily derivatized for coupling to a biological molecule (Bruchez et al., Science, 281:2013-2016, 1998). Similarly, highly fluorescent quantum dots (zinc sulfide-capped cadmium selenide) have been covalently coupled to biomolecules for use in ultrasensitive biological detection (Warren and Nie, Science, 281: 2016-2018, 1998).

Probe labeling can be performed, *e.g.,* during synthesis or, alternatively, post-synthetically, for example, using 5'-end labeling, which involves the enzymatic addition of a labeled nucleotide to the 5'-end of the probe using a terminal transferase. A single labeled nucleotide can be added by using a "chain terminating" nucleotide. Alternatively, non-terminating nucleotides can be used, resulting in multiple nucleotides being added to form a "tail." For synthesis labeling, labeled nucleotides (*e.g.,* phosphoramidite nucleotides) can be incorporated into the probe during chemical synthesis. Labels can be added to the 5', 3', or both ends of the probe (see, *e.g.,* U.S. Patent No. 5,082,830), or at base positions internal to the ODN.

Other methods for labeling nucleic acids utilize platinum-based labeling. Such methods include the Universal Linkage System (ULS, Kreatech Biotechnology B.V., Amsterdam, Netherlands). Platinum based labeling methods and their applications are described in, for example, U.S. Patent Nos. 5,580,990, 5,714,327, and 6,825,330; International Patent Publication Nos. WO 92/01699, WO 96/35696, and WO 98/15546; Hernandez-Santoset et al., Anal. Chem. 77:2868-2874, 2005; Raap et al., BioTechniques 37:1-6, 2004; Heetebrij et al., ChemBioChem 4:573-583, 2003; Van de Rijke et al., Analytical Biochemistry 321:71-78, 2003; Gupta et al., Nucleic Acids Research 31:e13, 2003; Van Gijlswijk et al., Clinical Chemistry 48:1352-1359, 2002; Alers et al., Genes, Chromosomes & Cancer 25:301-305, 1999; Wiegant et al., Cytogenetics and Cell Genetics 87:7-52, 1999; Jelsma et al., Journal of NIH Research 5:82, 1994; Van Belkum et al., BioTechniques 16:148-153, 1994; and Van Belkum et al., Journal of Virological Methods 45:189-200, 1993.

Labeled nucleotide(s) can also be attached to a probe using a crosslinker or a spacer. Crosslinkers may be homobifunctional or heterobifunctional. Suitable homobifunctional crosslinkers include, *e.g.,* amine reactive crosslinkers with NHS esters at each end (including, *e.g.,* dithiobis(succinimidylproponate) (DSP); 3, 3'-dithiobis(sulfosuccinimidylpropionate) (DTSSP); disuccinimidyl suberate (DSS); Bis(sulfosuccinimidyl)suberate (BS3); Ethylene glycolbis(succinimidylsuccinate) (EGS); Ethylene glycolbis(sulfosuccinimidylsuccinate) (SulfoEGS)); amine reactive crosslinkers with imidoesters at both ends (including, *e.g.,* dimethyl adipimidate (DMA); dimethyl pimelimidate (DMP); dimethyl suberimidate (DMS); dimethyl 3,3'-dithiobispropionimidate (DTBP)); sulfhydryl reactive crosslinkers with dithiopyridyl groups at each end (including, *e.g.,* 1,4-di-[3'-(2'-pyridyldithio)propionamdo]butane (DPDPB)); sulfhydryl reactive crosslinkers with maleimide groups at each end (including, *e.g.,* bismaleimidohexane (BMH)); carboxyl reactive crosslinkers with hydrazide groups at each end (including, e.g., adipic acid dihydrazide and carbonhydrazide); multi-group reactive crosslinkers with epoxide groups at each end (including, *e.g.,* 1,2:3,4-diepoxybutane; 1,2:5,6-diepoxyhexane; Bis(2,3-epoxypropyl)ether; 1,4-(butanediol) diglycidyl ether). Suitable heterobifunctional crosslinkers include crosslinkers with an amine reactive end and a sulfhydryl-reactive end (including, *e.g.,* N-Succinimidyl 3-(2-pyridyldithio)propionate (SPDP); long chain SPDP (SPDP); Sulfo-LC-SPDP; Succinimidyloxycarbonyl-α-methyl-α-(2-pyridydithio)toluene (SMPT); Sulfo-LC-SMPT; Succinimidyl-4-(N-maleimidomehyl)cyclohexane (SMCC); Sulfo-SMCC; Succinimidyl 6-((iodoacetyl)amino)hexanoate (SIAX); Succinimidyl 6-(6-(((4-iodoacetyl)amino)hexanoyl)amino)hexanoate (SIAXX)); crosslinkers with a carbonyl-reactive end and a sulfhydryl reactive end (including, *e.g.,* 4-(4-N-Maleimidophenyl)butyric acid hydrazide (MPBH); 4-(N-Maleimidomethyl)cyclohexane-1-carboxyl-hydrazide hydrochloride (M2C2H); 3-(2-Pyridyldithio)propinyl hydrazide (PDPH)); crosslinkers with an amine-reactive end and a photoreactive end (including, *e.g.,* Sulfosuccinimidyl-2-(p-azidosalicylicylamido)ethyl-1,3'-dithiopropionate (SASD); Sulfosuccinimidyl 2-(7-azido-4-methylcoumarin-3-acetamide)ethyl-1,3'- dithiopropionate (SAED)); crosslinkers with a sulfhydryl-reactive end and a photoreactive end (including, *e.g.,* N-[4-p-Azidosalicylamido)butyl]-3'-(2'pyridyldithio)propionamide (APDP)); crosslinkers with a carbonyl-reactive end and a photoreactive end (including, *e.g.,* 4-(p-Azidosalicylamido)butlyamine (ASBA)). Suitable spacers include, 5' ODN modifications such as dNTP's; and amine-reactive spacers such as amino- or sulfo-phosphoramidites including, *e.g.,* butylphosphoramidites, pentylphosphoramidites, hexylphosphoramidites, heptylphosphoramidites, octylphosphoramidites, nonylphosphoramidites, decylphosphoramidites, undecylphosphoramidites, dodecylphosphoramidites, pentadecylphosphoramidites, octadecylphosphoramidites. Other suitable amine-reactive spacers include *e.g.,* activated polyethylene glycol (PEG) such as (monomethoxy)n glycol, wherein n=3-18 unit repeats. Additional suitable crosslinkers and spacers are set forth in Herman. "Bioconjugate Chemistry". Academic Press. New York, NY. 1996.

### Washes, Counter-staining and Mounting

Typically, *in situ* hybridization techniques employ a series of successive wash steps following the hybridization step to remove unbound and/or non-specifically bound probe from the sample. Such wash steps can be performed in the isothermal methods of the invention. For example, following hybridization of probe to the sample, the hybridized sample can be washed in a solution of appropriate stringency to remove unbound and/or non-specifically bound probes. An appropriate stringency can be determined by washing the sample in successively higher stringency solutions and reading the signal intensity between each wash. Analysis of the data sets in this manner can reveal a wash stringency above which the hybridization pattern is not appreciably altered and which provides adequate signal for the particular probes of interest.

Suitable wash buffers for *in situ* hybridization methods are generally known in the art (See, *e.g.,* Sambrook and Russell, supra; Ausubel et al., supra. See also Tijssen, Laboratory Techniques in Biochemistry and Molecular Biology, Vol. 24: Hybridization with Nucleic Acid Probes (Elsevier, NY 1993)) and can include, for example, one or more salts (*e.g.,* sodium salts, lithium salts, potassium salts) and one or more detergents (*e.g.,* an ionic detergent, a non-ionic detergent). Suitable detergents for a wash buffer include, for example, sodium dodecyl sulfate (SDS), Triton^{®} X-100, Tween^{®} 20, NP-40, or Igepal CA-630. Preferably, the wash buffer comprises one or more salts (*e.g.,* sodium citrate) having a total concentration of about 0.03M to about 0.09M and about 0.1% SDS.

The number of washes and duration of each wash can be readily determined by one of ordinary skill in the art. Exemplary wash conditions for the isothermal methods of the invention include, for example, an initial post-hybridization wash in 2x SSC for 5 min. at room temperature (e.g, about 21° C) followed by one or more washes in 0.03M to 0.09M monovalent salt *(e.g.,* SSC) and 0.1% SDS at room temperature for at least about 2 minutes per wash, preferably, in the range of about 2 minutes to about 5 minutes per wash.

After the sample has been subjected to post-hybridization washes, chromosomal DNA in the sample is preferably counter-stained with a spectrally distinguishable DNA specific stain such as, for example, 4',6-diamidino-2-phenylindole (DAPI), propidium iodide (PI) or a Hoechst reagent/dye and mounted using an antifade reagent. The DNA stain can be added directly to the antifade reagent or can be incubated with the sample, drained and rinsed before the antifade reagent is added. Reagents and techniques for counterstaining and mounting samples are generally known in the art.

### Detection of Target Nucleic Acids

The isothermal methods of the invention further include detecting one or more target nucleic acids in the sample. The target nucleic acid is detected by detecting a labeled probe that has hybridized to the target nucleic acid. Detection of the probe label can be accomplished using an approach that is suitable for the particular label, which can be readily determine by those of ordinary skill in the art. For example, fluorophore labels can be detected by detecting the emission wavelength of the particular fluorophore used. Typical methods for detecting fluorescent signals include, *e.g.,* spectrofluorimetry, epifluorescence microscopy, confocal microscopy, and flow cytometry analysis. Fluorescent labels are generally preferred for detection of low levels of target because they provide a very strong signal with low background. Furthermore, fluorescent labels are optically detectable at high resolution and sensitivity through a quick scanning procedure, and different hybridization probes having fluorophores with different emission wavelengths (*e.g.,* fluorescein and rhodamine) can be used for a single sample to detect multiple target nucleic acids.

In the particular case of FISH procedures, which utilize fluorescent probes, a variety of different optical analyses can be utilized to detect hybridization complexes. Spectral detection methods are discussed, for example, in U.S. Patent No. 5,719,024; Schroeck et al. (Science 273:494-497, 1996); and Speicher et al. (Nature Genetics 12:368-375, 1996). Further guidance regarding general FISH procedures are discussed, for example, in Gall and Pardue (Methods in Enzymology 21:470-480, 1981); Henderson (International Review of Cytology 76:1-46, 1982); and Angerer et al. in Genetic Engineering: Principles and Methods (Setlow and Hollaender eds., Plenum Press, New York, 1985).

Detection of indirect labels typically involves detection of a binding partner, or secondary agent. For example, indirect labels such as biotin and other haptens (*e.g.,* digoxigenin (DIG), DNP, or fluorescein) can be detected via an interaction with streptavidin (*i.e.,* in the case of biotin) or an antibody as the secondary agent. Following binding of the probe and target, the target-probe complex can be detected by using, *e.g.,* directly labeled streptavidin or antibody. Alternatively, unlabeled secondary agents can be used with a directly labeled "tertiary" agent that specifically binds to the secondary agent (*e.g.,* unlabeled anti-DIG antibody can be used, which can be detected with a labeled second antibody specific for the species and class of the primary antibody). The label for the secondary agent is typically a non-isotopic label, although radioisotopic labels can be used. Typical non-isotopic labels include, *e.g.,* enzymes and fluorophores, which can be conjugated to the secondary or tertiary agent. Enzymes commonly used in DNA diagnostics include, for example, horseradish peroxidase and alkaline phosphatase.

Detection of enzyme labels can be accomplished, for example, by detecting color or dye deposition (*e.g.,* p-nitrophenyl phosphate or 5-bromo-4-chloro-3-indolyl phosphate/nitroblue tetrazolium for alkaline phosphatase and 3,3'-diaminobenzidine-NiCI2 for horseradish peroxidase), fluorescence (*e.g.,* 4-methyl umbelliferyl phosphate for alkaline phosphatase) or chemiluminescence (*e.g.,* the alkaline phosphatase dioxetane substrates LumiPhos 530 from Lumigen Inc., Detroit Mich. or AMPPD and CSPD from Tropix, Inc.), depending on the type of enzymatic label employed. Chemiluminescent detection can be carried out with X-ray or Polaroid film or by using single photon counting luminometers (*e.g.,* for alkaline phosphatase labeled probes).

In certain embodiments, digital enhancement or integration is used to detect a signal from a label on a probe. For example, detection of the label can include the use of microscopic imaging using a CCD camera mounted onto the eyepiece tube of a microscope (*e.g.,* a binocular, monocular, or stereo microscope). In some embodiments, detection of the label is accomplished using image scanning microscopy. For example, recent advances in computerized image scanning microscopy have significantly increased the ability to detect rare cells using fluorescence microscopy, permitting detection of 1 positive cell in an environment of ∼6x10⁵ negative cells (see, *e.g.,* Mehes et al., Cytometry 42:357-362, 2000). Advanced image scanning software has been developed that can not only detect multiple colors but also fused or co-localized signals useful for, *e.g.,* detection of translocations on the DNA level (MetaSystems Group, Inc.) Scanning speed typically depends on the number of parameters utilized for reliable detection of single positive cells. Image scanning also allows for images of the cells scored positive to be manually examined for confirmation. Advanced image scanning software for automated, slide-based analysis has been developed that can not only detect multiple colors but also fused or co-localized signals useful for, *e.g.,* detection of translocations on the DNA level (Meta System Group, Inc.) Scanning speed typically depends on the number of parameters utilized for reliable detection of single positive cells. Automated slide-based scanning systems are particularly amenable to high throughput assays.

In one embodiment, scanning slide microscopy, *e.g.,* employing a MetaCyte Automated Bio-Imaging System (Meta System Group, Inc.), is used. This system consists of the following components: 1) Carl Zeiss Axio Plan 2 MOT fluorescence microscope, 2) scanning 8-position stage, 3) PC Pentium III Processor, 4) Jai camera, 5) camera interface, 6) stage control, 7) trackball and mouse, and 8) printer. The focus analysis begins with a slide set-up loaded onto the microscope. The slide is scanned as the stage is moved and the image is captured. Following scanning of the entire slide, a gallery is created. Based on the criterion set up for positive or negative, the image analysis either results in a positive or negative signal. If negative, the slide is rescanned for rare event analyses. If positive, there is a filter change for the appropriate fluorescent signal and 5-7 planes are captured and analyzed. There is walk away/overnight operation for 8 slides (standard or 100 slides with optional tray changer). Adaptive detection algorithms and automatic exposure control function compensate for non-uniform staining conditions. Several markers can be detected simultaneously. The standard light source covers a wide spectrum from UV to IR. Scanning speed up to 1,000 cells per second can be used for rare cell detection if cellular fluorescent intensity allows detection in 1/1,000 sec. For strong signals, a lower magnification can be used to increase scanning speed.

Alternatively, detection of the probe can be performed in the absence of digital enhancement or integration.

### Kits for Detecting Target Nucleic Acids

In another embodiment, the invention relates to a kit for detecting a target nucleic acid (e.g., one or more target nucleic acids) in a biological sample under isothermal conditions; wherein the kit include at least one single-stranded oligonucleotide probe consisting of about 20 to about 50 nucleotides, wherein at least one detectable label is covalently attached; a denaturation buffer comprising NaOH about 0.03M to about 0.17M and about 50% to about 80% alcohol, a hybridization buffer comprising about 20% to about 90% formamide, dextran sulfate, and one or more salts at a final concentration of about 0.03M to about 0.09M; and a wash buffer that includes one or more salts at a final concentration of about 0.03M to about 0.09M, and about 0.1% SDS. In some embodiments, the kits may include additional, optional components, such as, for example, a secondary detection reagent, a stain for chromosomal DNA, an antifade reagent, instructions, protocols or a combination thereof. Typically, the kits are compartmentalized for ease of use and may include one or more containers with reagents. In one embodiment, all of the kit components are packaged together. Alternatively, one or more individual components of the kit may be provided in a separate package from the other kits components (e.g., the denaturation buffer may be packaged separately from the other kits components).

In one container, the kits of the invention include at least one single-stranded oligonucleotide probe that comprises a target binding region that is substantially complementary to a target sequence in a target nucleic acid. Preferably, each single-stranded oligonucleotide probe in the kits of the invention is a chromosome-specific probe. The single-stranded oligonucleotide probe typically consists of about 20 to about 50 nucleotides, preferably about 30 nucleotides. Suitable types of oligonucleotide probes (e.g, DNA, RNA, PNA) for use in the kits of the invention are described herein. Preferably, the oligonucleotide probes in the kits of the invention are DNA probes.

In certain embodiments, the single-stranded oligonucleotide probes consisting of about 20 to about 50 nucleotides in the kits of the invention are labeled (e.g., comprise one or more detectable labels). Exemplary detectable labels for single-stranded oligonucleotide probes are described herein. Preferably, the oligonucleotide probes in the kits of the invention comprise one or more fluorophores (e.g., fluorescein, rhodamine, Texas Red, phycoerythrin, Cy3, Cy5, Alexa 532, Alexa 546, Alexa 568, or Alexa 594).

In some embodiments, the kits of the invention include a plurality of different labeled probes, either mixed in the same container as a probe cocktail composition, or provided in separate containers. In such embodiments, each probe is specific for a particular target nucleic acid and comprises a detectable label that is distinguishable from the detectable labels present on other probes in the cocktail or kit that have specificity for different target nucleic acids. For example, each probe can comprise a fluorophore having a spectrally distinguishable emission wavelength. Suitable fluorophores for use in the kits of the invention having a plurality of different labeled probes include, e.g., Alexa 488 (excitation maximum at 492 nm and emission maximum at 520 nm) and Alexa 546 (excitation maximum at 555 nm and emission maximum at 570 nm)).

In a separate container, the kits of the invention include a denaturation buffer that comprises NaOH and about 50% to about 80% alcohol; wherein the denaturation buffer includes about 0.03N to about 0.17N sodium hydroxide, for example, about 0.05N, about 0.06N, about 0.07N, about 0.08N, about 0.09N or about 0.1N sodium hydroxide. Preferably, the denaturation buffer comprises about 0.07N NaOH (i.e., 0.07M NaOH). Exemplary alternative bases to NaOH not claimed herein for use in the kit's denaturation buffer include, for example, potassium hydroxide, barium hydroxide, caesium hydroxide, strontium hydroxide, calcium hydroxide, lithium hydroxide, rubidium hydroxide, magnesium hydroxide, butyl lithium, lithium diisopropylamide, lithium diethylamide, sodium amide, sodium hydride, lithium bis(trimethylsilyl)amide, sodium carbonate and ammonia, or a combination thereof. Preferably, the base is an alkali base. The denaturation buffer further includes at least one alcohol at a concentration of about 50% to about 80% by volume, for example about 60%, about 70% or about 80% by volume. Preferably, the alcohol is present at a concentration of about 70% by volume. Exemplary alcohols for use in the denaturation buffer include, for example, ethanol, methanol, propanol, butanol, pentanol and isoamyl alcohol, among others, or mixtures thereof. In a particular embodiment, the denaturation buffer comprises about 70% ethanol.

In another container, the kits of the invention include a hybridization buffer comprising about 20% to about 90% formamide, dextran sulfate, and one or more salts at a final concentration of about 0.03M to about 0.09M. Suitable concentrations of formamide for use in the hybridization buffer include about 60% to about 80% by volume (e.g., about 60%, about 65%, about 70%, about 75%, or about 80% by volume). The hybridization buffer may further include dextran sulfate (e.g., at a concentration of about 3% to about 20% by volume). In addition, the hybridization buffer may include one or more salts (e.g., sodium salts) at a final concentration of about 0.03M to about 0.09M. Preferably, the one or more salts include sodium citrate. Other suitable salts for use in the hybridization buffer include sodium chloride.

The kits of the invention further include one or more wash buffers that includes one or more salts (e.g., sodium salts, lithium salts or potassium salts) at a final concentration of about 0.03M to about 0.09M, and about 0.1% SDS. In a particular embodiment, the wash buffer includes sodium citrate and sodium chloride. In addition, the wash buffers in the kits of the invention may optionally include formamide.

Additional containers providing one or more reagent(s) for detecting the labeled probe can also be included in the kits of the invention. Such additional containers can include reagents or other elements recognized by the skilled artisan for use in a detection assay corresponding to the type of label on the probe. In one embodiment, the probes in the kit comprise an indirect label (*e.g.,* biotin) and the kit further includes at least a secondary agent for detecting the indirect label (*e.g.,* a container providing streptavidin labeled with a fluorophore).

A description of example embodiments of the invention follows.

### Example 1: Efficacy of a FISH procedure employing a room temperature denaturation step and standard, elevated temperature hybridization step

### Cytogenetic Slide Preparation

Human chromosome slides were prepared by harvesting peripheral blood cultures from an individual male donor. One mL peripheral blood per 25 mL culture flask from the donor was cultured in 10 mL RPMI 1640, 2 mM L-glutamine, FBS 10%, 250 µL PHA at 37°C and 5% CO₂. After 72 hours of culture, the cells were arrested in mitosis by adding 0.6 µl of colcemid (Karyomax, Invitrogen) per mL of culture. After 20min at 37°C, the cultures were centrifuged 10min at 1750rpm, the supernatant was discarded and 10mL of hypotonic solution, and 75mM KCl, was carefully added. After incubating for 20min at 37°C, several drops of Carnoy's fixative (Methanol:acetic acid, 3:1) were added to the tubes in order to perform a prefixation of the cells and facilitate further fixation without cell clumping. After centrifugation, cells were re-suspended in Carnoy's fixative. The fixation step was repeated 3 times until the pellets were clearly white. During the last fixation, the correct amount of fixative required for slide preparation was determined. Spreading was done at ∼22°C and ∼50% humidity on SuperFrost^{®} slides with one or two drops of cell suspension per slide. The slides were kept overnight at 37°C and then stored at -20°C in hermetic boxes with a desiccant until FISH was performed.

### Probes

X and Y Oligo-FISH™ probes (One Cell Systems, Inc., Cambridge, MA) were utilized. The probes were synthesized and labeled by Thermo Fisher Scientific (Ulm, Germany) using the DY fluors from Dyomics, GmbH (Jena, Germany). Chromosome X probe is labeled with the DY590 fluorescent dye and consists of 10 ODNs. The DXZ1 repeated 2 Kb sequence is present approximately 5,000 times in the pericentromeric region of human X chromosome (Yang 1982). This 2 Kb region consists of twelve 171-bp α-monomers arranged in imperfect direct repeats permitting X chromosome α-satellite repeat probes to be designed. To avoid cross hybridization to other chromosome α-satellite repeats, probes were designed to correspond to regions of the DXZ1 locus that have lower homology with the consensus α-monomer sequence. Chromosome Y probe consists of 4 ODNs and is labeled with the DY490 fluor. The DYZ1 region on Yq12 chromosome band consists of a 3.4kb sequence element present in 500 to 3000 copies. Throughout this repeat, high copy number TTCCA satellite 3 pentamer sequence repeats are interspersed among unique Y-chromosome specific sequence elements of varying length (Nakahori 1986; Weier 1990; Nakagome 1991). Ideally, for optimal hybridization, synthetic ODN Y-probes will be underrepresented for the TTCCA pentamer and will consist primarily of sequences comprised of approximately 50% CG-bases. 30mer ODN probes were designed for this region and compared to the human whole genome database (NCBI) using the Basic Local Alignment Tool (BLAST) (Altschul 1990). The sequences were compared to the non redundant genomic database (nr) with no filter.

### FISH Employing Room Temperature Denaturation and Conventional Hybridization Steps

Prepared cytogenetic slides harvested from human peripheral blood were denatured in a solution of NaOH in 70% ethanol at 21°C for varying denaturation times ranging from 3 min. to 20 min. Different concentrations of NaOH ranging from 0.03M to 0.17M were tested. The slides were then dehydrated by an ethanol gradient (80%, 90%, and 100%) for 2 min each and air dried. Equal volumes of hybridization buffer and probe cocktail were mixed to obtain the hybridization mix used in this procedure. Cocktails were used in a working volume of 10µL. The area of interest on each slide was located with a phase contrast microscope and a 10 µL volume of Oligo-FISH™ X, Yq12 cocktail was dropped on the slide and covered with a 22mm x 22mm coverslip. The hybridization was carried out at 37°C for 5 min. After hybridization, the slides were washed in 2x SSC under agitation to remove the coverslip and then washed (0.2xSSC, 0.1% SDS) at 50°C for 2 min with agitation for 30sec. Finally, slides were collected in 2xSSC, mounted with antifade with DAPI and covered with a 50mm x 22mm cover slip (#1 thickness). FISH data using the average signal-to-noise ratio (SNR) taken from 50 interphase nuclei for each probe were compared..

### Determination of Signal Intensity

Signal intensity was determined by the signal-to-noise ratio (SNR). Using NIS-Elements software, FISH images from 50 interphase nuclei (minimal number required for statistical analysis), acquired under identical conditions, were segmented by the threshold value of the gray level to differentiate between signal and cell nucleus. For each cell, the gray level mean, defined as the sum of gray levels in the measured segment, divided by the segment area in pixels, and standard deviation were calculated. Signal to noise ratio was then determined as the signal mean gray level divided by the background mean gray level.

### Fluorescence Microscopy and Image Acquisition

Fluorescence microscopy analysis and digital image capture were performed using a Nikon Eclipse 90i microscope (Nikon Instruments, Melville, NY) equipped with a CoolSNAP™ HQ2 CCD camera (Photometries Ltd., Tucson, AZ). Images were captured and measured using Nikon NIS-Elements software.

### Results

Of the different NaOH concentrations tested, 0.07M NaOH in 70% ethanol gave the highest SNR. In addition, 15 min. was found to be the optimal denaturation time. Figure 1 shows that isothermal denaturation for 15 min. produced statistically similar SNRs for X and Y probes compared to conventional denaturation (70% formamide at 72°C), when conventional hybridization (37°C) and wash (50°C) temperatures were employed for both procedures.

### Example 2: Efficacy of a FISH procedure employing room temparature denaturation and hybridization steps

### FISH Employing Isothermal Denaturation and Hybridization Steps

Prepared cytogenetic slides harvested from human peripheral blood (described in Example 1) were denatured in 0.07M NaOH in 70% ethanol at 21 °C for 15 min. The slides were then dehydrated by an ethanol gradient (80%, 90%, and 100%) for 2 min each and air dried. The area of interest on each slide was located with a phase contrast microscope and a 10 µL volume of Oligo-FISH™ X, Yq12 cocktail was dropped on the slide and covered with a 22mm x 22mm coverslip. The hybridization was carried out at room temperature (about 21°C) for 10 min. After hybridization, the slides were washed in 2x SSC for 5 min. under agitation to remove the coverslip. After the isothermal denaturation and hybridization, the slides were washed in 0.09M monovalent salt (SSC) and 0.1% sodium dodecyl sulfate (SDS) at room temperature. Finally, slides were collected in 2xSSC, mounted with antifade with DAPI and covered with a 50mm x 22mm cover slip (#1 thickness). FISH data using the average signal-to-noise ratio (SNR) taken from 50 interphase nuclei for each probe were compared.

Cytogenetic slide preparation, probes, determination of signal intensity and fluorescence microscopy and image acquisition were performed as generally described in Example 1.

### Results

After establishing optimal conditions for room temperature denaturation, a room temperature hybridization condition (21 ° C hybridization, 5 min.) was tested using the same hybridization buffer used for conventional FISH described in Example 1, combined with pre-treatment/denaturation in 0.07M NaOH / 70% ethanol for 15 min. at room temperature. The same Oligo-FISH™ X and Y probe set and conventional wash conditions (0.2X SSC, 0.1% SDS, 50°C) employed in Example 1 herein were used. Isothermal hybridization images are shown in Figure 2. FISH signals for X (red) and Y (green) probes are clearly seen in interphase nuclei, as well as on the corresponding chromosomes in the metaphase spread.

## Claims

1. A method for determining whether a target nucleic acid is present in a biological sample on a solid support, comprising the steps of:
a) contacting the sample on the support with a denaturation buffer comprising a base and about 50% to about 80% alcohol, wherein the base is present in the solution at a concentration of about 0.03N to about 0.17N, thereby denaturing the sample;
b) incubating the denatured sample in a hybridization buffer that comprises at least one single-stranded oligonucleotide probe at a temperature in the range of about 19 degrees Celsius to about 25 degrees Celsius, wherein the oligonucleotide probe comprises a nucleotide sequence that is substantially complementary to a nucleotide sequence in the target nucleic acid and at least one detectable label;
c) washing the sample to remove unhybridised oligonucleotide probes; and
d) determining whether the target nucleic acid is present in the sample by detecting one or more oligonucleotide probes that have hybridized to the target nucleic acid in the sample.

2. The method of Claim 1, wherein the base is sodium hydroxide.

3. The method of Claim 1 or 2, wherein step a) is performed at a temperature of about 19 degrees Celsius to about 25 degrees Celsius, for example at a temperature of about 21 degrees Celsius.

4. The method of Claim 3, wherein the sample is contacted with the solution for about 3 to about 20 minutes in step a), for example for about 11 to about 17 minutes in step a), and preferably for about 13 to about 15 minutes in step a).

5. The method of Claim 3, wherein the solution in step a) comprises about 0.07M sodium hydroxide.

6. The method of Claim 5, wherein the solution in step a) comprises about 70% ethanol.

7. The method of Claim 1 or 2, wherein step b) is performed at a temperature of about 21 degrees Celsius.

8. The method of Claim 1, wherein, prior to step b), the oligonucleotide probe is in a hybridization buffer that includes formamide, dextran sulfate, and one or more salts at a final concentration of about 0.03M to about 0.09M.

9. The method of Claim 1, wherein the step of removing unhybridised oligonucleotide probes from the sample comprises washing the sample in a wash buffer at a temperature of about 19 degrees Celsius to about 25 degrees Celsius prior to step c).

10. The method of Claim 9, wherein the wash buffer includes one or more salts at a final concentration of about 0.03M to about 0.09M, and sodium dodecyl sulfate (SDS).

11. The method of Claim 1, wherein the oligonucleotide probe comprises about 20 to about 50 nucleotides, for example about 30 nucleotides.

12. The method of Claim 11, wherein the oligonucleotide probe is a synthetic oligonucleotide probe.

13. The method of Claim 1, wherein the at least one detectable label is attached to the oligonucleotide by a covalent bond.

14. The method of Claim 13, wherein the at least one detectable label comprises a fluorescent label.

15. A method for detecting a target nucleic acid in a biological sample on a solid support, comprising the steps of:
a) contacting the sample on the support with a denaturation buffer comprising a base and about 50% to about 80% alcohol, wherein the base is present in the solution at a concentration of about 0.03N to about 0.17N, thereby denaturing the sample;
b) hybridizing at least one single-stranded oligonucleotide probe to the target nucleic acid in the denatured sample in a hybridization buffer at a temperature in the range of about 19 degrees Celsius to about 25 degrees Celsius, wherein the oligonucleotide probe comprises a nucleotide sequence that is substantially complementary to a nucleotide sequence in the target nucleic acid and at least one detectable label;
c) washing the sample to remove unhybridised oligonucleotide probes; and
d) detecting the at least one detectable label on the oligonucleotide probe following hybridization to the target nucleic acid in the sample, thereby detecting the target nucleic acid in the sample.

16. The method of Claim 15, wherein the base is sodium hydroxide.

17. The method of Claim 1 or 15, wherein the biological sample comprises urothelial cells.

18. A kit for detecting a target nucleic acid in a biological sample, comprising:
a) at least one single-stranded oligonucleotide probe consisting of about 20 to about 50 nucleotides, wherein at least one detectable label is covalently attached to the oligonucleotide probe;
b) a denaturation buffer comprising about 0.03M to about 0.17M sodium hydroxide and about 50% to about 80% alcohol;
c) a hybridization buffer comprising about 20% to about 90% formamide, dextran sulfate, and one or more salts at a final concentration of about 0.03M to about 0.09M; and
d) a wash buffer that includes one or more salts at a final concentration of about 0.03M to about 0.09M, and about 0.1 % SDS.

19. The kit of Claim 18, wherein the denaturation buffer includes about 0.07M sodium hydroxide and about 70% ethanol.

20. The kit of Claim 18 or 19, wherein the hybridization buffer includes about 60% to about 80% formamide.

21. The kit of Claim 18, wherein the one or more salts in the wash buffer are selected from the group consisting of a sodium salt, a lithium salt and a potassium salt.

22. The kit of Claim 18, wherein the one or more salts in the wash buffer include sodium citrate and sodium chloride.

23. The kit of Claim 21 or 22, wherein the wash buffer further includes formamide.

24. A method for detecting a target nucleic acid in a biological sample on a solid support, comprising the steps of:
a) contacting the sample on the support with a denaturation buffer comprising about 0.07M sodium hydroxide and about 70% ethanol for about 13 to about 15 minutes at a temperature in the range of about 19 degrees Celsius to about 25 degrees Celsius, thereby denaturing the sample;
b) hybridizing at least one single-stranded oligonucleotide probe consisting of about 20 to about 50 nucleotides to the target nucleic acid in the denatured sample in a hybridization buffer at a temperature in the range of about 19 degrees Celsius to about 25 degrees Celsius, wherein the oligonucleotide probe comprises a nucleotide sequence that is substantially complementary to a nucleotide sequence in the target nucleic acid, and at least one fluorescent detectable label covalently attached to the oligonucleotide probe;
c) washing the sample to remove unhybridised oligonucleotide probes; and
d) detecting the fluorescent detectable label on the oligonucleotide probe, thereby detecting the target nucleic acid in the sample.

## Patentansprüche

1. Verfahren zur Bestimmung, ob eine Targetnukleinsäure in einer biologischen Probe auf einem festen Träger vorliegt, wobei das Verfahren die folgenden Schritte umfasst:
a) In-Kontakt-bringen der Probe auf dem Träger mit einem Denaturierungspuffer, der eine Base und etwa 50% bis etwa 80% Alkohol umfasst, wobei die Base in der Lösung in einer Konzentration von etwa 0,03N bis etwa 0,17N vorliegt, wodurch die Probe denaturiert wird;
b) Inkubieren der denaturierten Probe in einem Hybridisierungspuffer, der wenigstens eine einsträngige Oligonukleotidsonde umfasst, bei einer Temperatur im Bereich von etwa 19°C bis etwa 25°C, wobei die Oligonukleotidsonde eine Nukleotidsequenz, die im Wesentlichen komplementär zu einer Nukleotidsequenz in der Targetnukleinsäure ist, sowie wenigstens eine erfassbare Markierung umfasst;
c) Waschen der Probe, um die nicht hybridisierten Oligonukleotidsonden zu entfernen; und
d) Bestimmen, ob die Targetnukleinsäure in der Probe vorliegt, durch Erfassen einer oder mehrere Oligonukleotidsonden, die in der Probe zur Targetnukleinsäure hybridisierten.

2. Verfahren nach Anspruch 1, wobei die Base Natriumhydroxid ist.

3. Verfahren nach Anspruch 1 oder 2, wobei Schritt a) bei einer Temperatur von etwa 19°C bis etwa 25°C Celsius durchgeführt wird, zum Beispiel bei einer Temperatur von etwa 21°C.

4. Verfahren nach Anspruch 3, wobei die Probe etwa 3 bis etwa 20 Minuten in Schritt a) mit der Lösung in Kontakt gebracht wird, zum Beispiel etwa 11 bis etwa 17 Minuten in Schritt a), und bevorzugt etwa 13 bis etwa 15 Minuten in Schritt a).

5. Verfahren nach Anspruch 3, wobei die Lösung in Schritt a) etwa 0,07M Natriumhydroxid umfasst.

6. Verfahren nach Anspruch 5, wobei die Lösung in Schritt a) etwa 70% Ethanol umfasst.

7. Verfahren nach Anspruch 1 oder 2, wobei Schritt b) bei einer Temperatur von etwa 21°C durchgeführt wird.

8. Verfahren nach Anspruch 1, wobei, vor Schritt b), die Oligonukleotidsonde ein Hybridisierungspuffer ist, der Formamid, Dextransulfat, und ein Salz oder mehrere Salze bei einer Endkonzentration von etwa 0,03M bis etwa 0,09M umfasst.

9. Verfahren nach Anspruch 1, wobei der Schritt des Entfernens nicht hybridisierter Oligonukleotidsonden von der Probe das Waschen der Probe in einem Waschpuffer bei einer Temperatur von etwa 19°C bis etwa 25°C vor Schritt c) umfasst.

10. Verfahren nach Anspruch 9, wobei der Waschpuffer ein Salz oder mehrerer Salze in einer Endkonzentration von etwa 0,03M bis etwa 0,09M, und Natriumdodecylsulfat (sodium dodecyl sulfate, SDS) umfasst.

11. Verfahren nach Anspruch 1, wobei die Oligonukleotidsonde etwa 20 bis etwa 50 Nukleotide umfasst, zum Beispiel etwa 30 Nukleotide.

12. Verfahren nach Anspruch 11, wobei die Oligonukleotidsonde eine synthetische Oligonukleotidsonde ist.

13. Verfahren nach Anspruch 1, wobei die wenigstens eine erfassbare Markierung durch eine kovalente Bindung am Oligonukleotid angebracht ist.

14. Verfahren nach Anspruch 13, wobei die wenigstens eine erfassbare Markierung eine Fluoreszenzmarkierung umfasst.

15. Verfahren zum Erfassen einer Targetnukleinsäure in einer biologischen Probe auf einem festen Träger, wobei das Verfahren die folgenden Schritte umfasst:
a) In-Kontakt-bringen der Probe auf dem Träger mit einem Denaturierungspuffer, der eine Base und etwa 50% bis etwa 80% Alkohol umfasst, wobei die Base in der Lösung in einer Konzentration von etwa 0,03N bis etwa 0,17N vorliegt, wodurch die Probe denaturiert wird;
b) Hybridisieren wenigstens einer einsträngigen Oligonukleotidsonde zur Targetnukleinsäure in der denaturierten Probe in einem Hybridisierungspuffer bei einer Temperatur im Bereich von etwa 19°C bis etwa 25°C, wobei die Oligonukleotidsonde eine Nukleotidsequenz, die im Wesentlichen komplementär zu einer Nukleotidsequenz in der Targetnukleinsäure ist, sowie wenigstens eine erfassbare Markierung umfasst;
c) Waschen der Probe, um die nicht hybridisierten Oligonukleotidsonden zu entfernen; und
d) Erfassen der wenigstens einen erfassbaren Markierung auf der Oligonukleotidsonde nach der Hybridisierung zur Targetnukleinsäure in der Probe, wobei die Targetnukleinsäure in der Probe erfasst wird.

16. Verfahren nach Anspruch 15, wobei die Base Natriumhydroxid ist.

17. Verfahren nach Anspruch 1 oder 15, wobei die biologische Probe Urothelzellen umfasst.

18. Kit zum Erfassen einer Targetnukleinsäure in einer biologischen Probe, umfassend:
a) wenigstens eine einsträngige Oligonukleotidsonde bestehend aus etwa 20 bis etwa 50 Nukleotiden, wobei wenigstens eine erfassbare Markierung kovalent an der Oligonukleotidsonde angebracht ist;
b) einen Denaturierungspuffer, der etwa 0,03M bis etwa 0,17M Natriumhydroxid und etwa 50% bis etwa 80% Alkohol umfasst;
c) einen Hybridisierungspuffer, der etwa 20% bis etwa 90% Formamid, Dextransulfat, und ein Salz oder mehrere Salze in einer Endkonzentration von etwa 0,03M bis etwa 0,09M umfasst; und
d) einen Waschpuffer, der ein Salz oder mehrere Salze in einer Endkonzentration von etwa 0,03M bis etwa 0,09M umfasst, sowie etwa 0,1% SDS.

19. Kit nach Anspruch 18, wobei der Denaturierungspuffer etwa 0,07M Natriumhydroxid und etwa 70% Ethanol umfasst.

20. Kit nach Anspruch 18 oder 19, wobei der Hybridisierungspuffer etwa 60% bis etwa 80% Formamid umfasst.

21. Kit nach Anspruch 18, wobei das eine Salz oder die mehreren Salze in dem Waschpuffer ausgewählt sind aus der Gruppe bestehend aus Natriumsalz, Lithiumsalz und Kaliumsalz.

22. Kit nach Anspruch 18, wobei das eine Salz oder die mehreren Salze in dem Waschpuffer Natriumcitrat und Natriumchlorid umfassen.

23. Kit nach Anspruch 21 oder 22, wobei der Waschpuffer ferner Formamid umfasst.

24. Verfahren zur Erfassung einer Targetnukleinsäure in einer biologischen Probe auf einem festen Träger, wobei das Verfahren die folgenden Schritte umfasst:
a) In-Kontakt-bringen der Probe auf dem Träger mit einem Denaturierungspuffer, der etwa 0,07M Natriumhydroxid und etwa 70% Ethanol umfasst, für etwa 13 bis etwa 15 Minuten bei einer Temperatur im Bereich von etwa 19°C bis etwa 25°C, wodurch die Probe denaturiert wird;
b) Hybridisieren wenigstens einer einsträngigen Oligonukleotidsonde, die aus etwa 20 bis etwa 50 Nukleotiden besteht, zur Targetnukleinsäure in der denaturierten Probe in einem Hybridisierungspuffer bei einer Temperatur im Bereich von etwa 19°C bis etwa 25°C, wobei die Oligonukleotidsonde eine Nukleotidsequenz, die im Wesentlichen komplementär zu einer Nukleotidsequenz in der Targetnukleinsäure ist, sowie wenigstens ein erfassbare Fluoreszenzmarkierung umfasst, welche kovalent an der Oligonukleotidsonde angebracht ist;
c) Waschen der Probe, um die nicht hybridisierten Oligonukleotidsonden zu entfernen; und
d) Erfassen der erfassbaren Fluoreszenzmarkierung auf der Oligonukleotidsonde, wobei die Targetnukleinsäure in der Probe erfasst wird.

## Revendications

1. Procédé permettant de déterminer si un acide nucléique cible est présent dans un échantillon biologique sur un support solide, comprenant les étapes suivantes :
a) la mise en contact de l'échantillon sur le support avec un tampon de dénaturation comprenant une base et environ 50 % à environ 80 % d'alcool, où la base est présente dans la solution à une concentration d'environ 0,03 N à environ 0,17 N, dénaturant de cette manière l'échantillon ;
b) l'incubation de l'échantillon dénaturé dans un tampon d'hybridation qui comprend au moins une sonde oligonucléotidique simple brin à une température dans la plage d'environ 19 degrés Celsius à environ 25 degrés Celsius, où la sonde oligonucléotidique comprend une séquence nucléotidique qui est substantiellement complémentaire d'une séquence nucléotidique dans l'acide nucléique cible et au moins un marqueur détectable ;
c) le lavage de l'échantillon pour éliminer les sondes oligonucléotidiques non hybridées ; et
d) la détermination si l'acide nucléique cible est présent dans l'échantillon par la détection d'une ou de plusieurs sondes oligonucléotidiques qui se sont hybridées à l'acide nucléique cible dans l'échantillon.

2. Procédé selon la revendication 1, dans lequel la base est l'hydroxyde de sodium.

3. Procédé selon la revendication 1 ou 2, dans lequel l'étape a) est réalisée à une température d'environ 19 degrés Celsius à environ 25 degrés Celsius, par exemple à une température d'environ 21 degrés Celsius.

4. Procédé selon la revendication 3, dans lequel l'échantillon est mis en contact avec la solution pendant environ 3 à environ 20 minutes dans l'étape a), par exemple pendant environ 11 à environ 17 minutes dans l'étape a), et de préférence pendant environ 13 à environ 15 minutes dans l'étape a).

5. Procédé selon la revendication 3, dans lequel la solution dans l'étape a) comprend environ 0,07 M d'hydroxyde de sodium.

6. Procédé selon la revendication 5, dans lequel la solution dans l'étape a) comprend environ 70 % d'éthanol.

7. Procédé selon la revendication 1 ou 2, dans lequel l'étape b) est réalisée à une température d'environ 21 degrés Celsius.

8. Procédé selon la revendication 1, dans lequel, avant l'étape b), la sonde oligonucléotidique est dans un tampon d'hybridation qui comprend du formamide, du sulfate de dextrane, et un ou plusieurs sels à une concentration finale d'environ 0,03 M à environ 0,09 M.

9. Procédé selon la revendication 1, dans lequel l'étape d'élimination des sondes oligonucléotidiques non hybridées à partir de l'échantillon comprend le lavage de l'échantillon dans un tampon de lavage à une température d'environ 19 degrés Celsius à environ 25 degrés Celsius avant l'étape c).

10. Procédé selon la revendication 9, dans lequel le tampon de lavage comprend un ou plusieurs sels à une concentration finale d'environ 0,03 M à environ 0,09 M, et du dodécylsulfate de sodium (SDS).

11. Procédé selon la revendication 1, dans lequel la sonde oligonucléotidique comprend environ 20 à environ 50 nucléotides, par exemple environ 30 nucléotides.

12. Procédé selon la revendication 11, dans lequel la sonde oligonucléotidique est une sonde oligonucléotidique synthétique.

13. Procédé selon la revendication 1, dans lequel le au moins un marqueur détectable est fixé à l'oligonucléotide par une liaison covalente.

14. Procédé selon la revendication 13, dans lequel le au moins un marqueur détectable comprend un marqueur fluorescent.

15. Procédé de détection d'un acide nucléique cible dans un échantillon biologique sur un support solide, comprenant les étapes suivantes :
a) la mise en contact de l'échantillon sur le support avec un tampon de dénaturation comprenant une base et environ 50 % à environ 80 % d'alcool, où la base est présente dans la solution à une concentration d'environ 0,03 N à environ 0,17 N, dénaturant de cette manière l'échantillon ;
b) l'hybridation d'au moins une sonde oligonucléotidique simple brin à l'acide nucléique cible dans l'échantillon dénaturé dans un tampon d'hybridation à une température dans la plage d'environ 19 degrés Celsius à environ 25 degrés Celsius, où la sonde oligonucléotidique comprend une séquence nucléotidique qui est substantiellement complémentaire d'une séquence nucléotidique dans l'acide nucléique cible et au moins un marqueur détectable ;
c) le lavage de l'échantillon pour éliminer les sondes oligonucléotidiques non hybridées ; et
d) la détection du au moins un marqueur détectable sur la sonde oligonucléotidique à la suite de l'hybridation à l'acide nucléique cible dans l'échantillon, détectant de cette manière l'acide nucléique cible dans l'échantillon.

16. Procédé selon la revendication 15, dans lequel la base est l'hydroxyde de sodium.

17. Procédé selon la revendication 1 ou 15, dans lequel l'échantillon biologique comprend des cellules urothéliales.

18. Kit pour la détection d'un acide nucléique cible dans un échantillon biologique, comprenant :
a) au moins une sonde oligonucléotidique simple brin constituée d'environ 20 à environ 50 nucléotides, où au moins un marqueur détectable est fixé de manière covalente à la sonde oligonucléotidique ;
b) un tampon de dénaturation comprenant environ 0,03 M à environ 0,17 M d'hydroxyde de sodium et environ 50 % à environ 80 % d'alcool ;
c) un tampon d'hybridation comprenant environ 20 % à environ 90 % de formamide, du sulfate de dextrane, et un ou plusieurs sels à une concentration finale d'environ 0,03 M à environ 0,09 M ; et
d) un tampon de lavage qui comprend un ou plusieurs sels à une concentration finale d'environ 0,03 M à environ 0,09 M, et environ 0,1 % de SDS.

19. Kit selon la revendication 18, dans lequel le tampon de dénaturation comprend environ 0,07 M d'hydroxyde de sodium et environ 70 % d'éthanol.

20. Kit selon la revendication 18 ou 19, dans lequel le tampon d'hybridation comprend environ 60 % à environ 80 % de formamide.

21. Kit selon la revendication 18, dans lequel les un ou plusieurs sels dans le tampon de lavage sont choisis dans le groupe constitué d'un sel de sodium, d'un sel de lithium et d'un sel de potassium.

22. Kit selon la revendication 18, dans lequel les un ou plusieurs sels dans le tampon de lavage comprennent le citrate de sodium et le chlorure de sodium.

23. Kit selon la revendication 21 ou 22, dans lequel le tampon de lavage comprend en outre du formamide.

24. Procédé de détection d'un acide nucléique cible dans un échantillon biologique sur un support solide, comprenant les étapes suivantes :
a) la mise en contact de l'échantillon sur le support avec un tampon de dénaturation comprenant environ 0,07 M d'hydroxyde de sodium et environ 70 % d'éthanol pendant environ 13 à environ 15 minutes à une température dans la plage d'environ 19 degrés Celsius à environ 25 degrés Celsius, dénaturant de cette manière l'échantillon ;
b) l'hybridation d'au moins une sonde oligonucléotidique simple brin constituée d'environ 20 à environ 50 nucléotides à l'acide nucléique cible dans l'échantillon dénaturé dans un tampon d'hybridation à une température dans la plage d'environ 19 degrés Celsius à environ 25 degrés Celsius, où la sonde oligonucléotidique comprend une séquence nucléotidique qui est substantiellement complémentaire d'une séquence nucléotidique dans l'acide nucléique cible et au moins un marqueur détectable fluorescent fixé de manière covalente à la sonde oligonucléotidique ;
c) le lavage de l'échantillon pour éliminer les sondes oligonucléotidiques non hybridées ; et
d) la détection du marqueur détectable fluorescent sur la sonde oligonucléotidique, détectant de cette manière l'acide nucléique cible dans l'échantillon.
